# EUROPEAN PATENT SPECIFICATION

(11) **EP 1 692 296 B1**
(45) Date of publication and mention of the grant of the patent: **15.04.2009**
(21) Application number: 03772434.1
(22) Date of filing: 07.11.2003
(51) Int. Cl.: C12P 1/06, C12N 9/62

(54) **A PROCESS FOR PRODUCING AN ALKALINE PROTEASE FROM A DEEP-SEA FUNGUS**
VERFAHREN ZUR HERSTELLUNG EINER ALKALISCHEN PROTEASE AUS EINEM TIEFSEEPILZ
PROCEDE DE PRODUCTION D'UNE PROTEASE ALCALINE A PARTIR D'UN CHAMPIGNON PELAGIQUE

(43) Date of publication of application: 23.08.2006
(73) Proprietor: Council of Scientific and Industrial Research, New Delhi 110 001 (IN)
(72) Inventor: RAGHUKUMAR, Chandralata, 403 004 Goa (IN); DAMARE, Samir, Ravikant, 403 004 Goa (IN); MURALEEDHARAN, Usha, Devi, Taleigna Plateau, 403 206 Goa (IN)
(74) Representative: Wilson Gunn
(86) International application number: PCT/IB2003/005000
(87) International publication number: WO 2005/045047

(56) References cited:
- WO-A-92/17576
- WO-A-98/40473
- US-A- 5 474 700
- PATENT ABSTRACTS OF JAPAN vol. 018, no. 043 (C-1156), 24 January 1994 (1994-01-24) -& JP 05 268954 A (HOKKAIDO TOGYO KK), 19 October 1993 (1993-10-19)
- PATENT ABSTRACTS OF JAPAN vol. 1997, no. 04, 30 April 1997 (1997-04-30) -& JP 08 322562 A (KAO CORP), 10 December 1996 (1996-12-10)
- PATENT ABSTRACTS OF JAPAN vol. 1998, no. 03, 27 February 1998 (1998-02-27) & JP 09 299084 A (KAO CORP), 25 November 1997 (1997-11-25)

## Description

### FIELD OF PRESENT INVENTION

Present invention relates to a process for production of protease enzyme, using a marine strain of the fungus *Aspergillus* sp., isolated from deep-sea sediments collected at a depth of ∼5000m in the Central Indian basin and deposited in the microbial type culture collection (MTCC) of Institute of Microbial Technology, Chandigarh, India, under the accession number MTCC 5102. Furthermore, the protease produced by the said fungus *Aspergillus* sp. MTCC 5102 is active at pH 6-11 and temperature of 15°C to 60°C and optimum at 45°C. One hundred percent of the enzyme activity is retained after incubating the enzyme at 42-47°C for about 30 minutes and about 90% of the activity is retained after 45 minutes of incubation at 45°C.

### BACKGROUND OF THE PRESENT INVENTION

One of the major applications of alkaline proteases is in the laundry detergents. "Enzyme detergents" enable to remove proteinaceous material from stains. Environmentally friendly non-phosphate detergents are effective due to the presence of such active enzymes in their formulations. Alkaline protease is also used in dishwashing detergents and in cleaning of ultrafiltration and reverse osmosis membranes in modem dairy and food industries. In addition, contact lens cleaning solutions also contain alkaline protease. Alkaline protease is also used in dehairing of skin and hides. Traditional methods use sodium sulfite for this purpose which contributes 100% of the sulfide and about 80% of the suspended solids in tannery effluents. Alkaline protease finds its application in silver recovery from gelatine-coated X-ray films. Silver is recovered conventionally from X-ray films by burning, which causes environmental pollution. Therefore, hydrolysis of gelatin using alkaline protease is an environmentally friendly process. Alkaline protease is also used in food industries for hydrolysis of plant, fish and animal material. Alkaline proteases find use in treatment of industrial and domestic waste and processing of waste feathers from poultry slaughter houses.

Attempts to use fungi or protease systems from fungi and bacteria for various applications as mentioned above are reported in literature. Microbial alkaline proteases (subtilisins: E.C. 3.4.21.14) are a commercially important group of enzymes for detergent industries. Ideally, proteases used in detergent industries should have high activity and stability over a broad range of pH and temperature.

Thus, various organisms have been used for production of alkaline protease for use in detergent industry (Takami, H., Akiba, T. and Horikoshi, K. 1990. Characterization of an alkaline protease from Bacillus sp. No. AH 101. Applied Microbiology and Biotechnology 33: 519-523).
(i) A reference may be made to a world patent no. WO 8803948 wherein, the fungus *Paecilomyces marquandii* (NRRL 18048) grown in a medium containing suitable carbon and nitrogen source and pH adjusted to 9.2 produced alkaline protease having optimum activity at 45°C and pH range of 7-10.5 (Beck et al., 1988. Low-temperature active alkaline protease from *Paecilomyces marquandii* and its preparation. Patent No. WO 8803948). Our culture shows optimum activity at 45°C but also shows near optimum activity (90%) at 30°C and 50% activity at 15°C and 60°C and 90-100% of the activity is shown at a pH range of 6-11. The fungus cultured at pH 7.0 or 8.5 produce protease active at pH 6-11. The fungus can be cultured using sea water instead of distilled water and also be incubated at 5°C for growth.
(ii) A reference may be made to a world patent no. WO 8803947 wherein, an actinomycete culture *Nocardiopsis dassonvillei* strain N58-1 was used to prepare a low temperature active alkaline protease. The actinomycete was grown on suitable carbon and nitrogen source at 8 - 10 % concentration at alkaline pH values and in the temperature range of 20 - 30°C. However, the optimum temperature for protease activity was at 60°C and at pH of 8 - 9 (Liu et al 1988, Low temperature active alkaline protease from *Nocandiopsis dassonvil*/*ei* and its preparation, Patent No. WO 8803947). Our culture shows optimum activity at 45°C but also shows near optimum activity (90%) at 30°C and 50% activity at 15°C and 60°C and 90-100% of the activity is shown at a pH range of 6-11. The fungus cultured at pH 7.0 or 8.5 produces protease active at pH 6-11. The fungus can be cultured using sea water instead of distilled water and shows reasonably good growth at 5°C.
(iii) A reference may be made to a publication wherein, a marine bacterium *Alteromonas haloplanktis* strain S5b showed optimum activity between pH 8 and 9 and optimum temperature for the activity was 20°C (Suzuki and Odagami. 1997. Low temperature-active thiol protease from marine bacterium Alteromonas haloplanktis. Journal of Marine Biotechnology, 5:230-233). However, the protease shows a very narrow temperature range for its activity. Its optimum activity is at 20°C which drops down to less than 20 % at 30°C thus appears to be extremely heat labile. The protease activity in our culture NIOCC 20 shows almost similar activity at 30°C and 45°C (90 % and 100 %, respectively).
**(iv)** A reference may be made to a publication wherein, *Rhizopus oryzae* strain RO IIT KGP produced an extracellular alkaline serine protease with an optimum pH of 5 at 30°C and pH of 10 at 37°C. The enzyme was stable between pH 3 - 11 and the optimum temperature was 60°C (Banerjee and Bhattacharya, 1992. Extracellular alkaline protease of newly isolated Rhizopus oryzae. Biotechnology Letters, 14: 301-304). Our culture however produced protease with optimum activity between 30 and 45°C and at a broad pH range of 6 - 11.
**(v)** A reference may be made to a publication wherein, *Azospirillum* spp. Isolated from a mountain soil sample in Korea showed protease activity in a broad pH range around 8.5 and temperature up to 45°C (Kun-Hee et al, 1999. Isolation of psychrotrophic Azospirillum sp. and characterization of its extracellular protease. FEMS Microbiology Letters, 174: 173 - 178). Protease was active as well as stable at low temperature below 30°C, but it was inhibited in the presence of EDTA. However, the protease produced by our culture NIOCC 20 was not inhibited by EDTA.
**(vi)** A reference may be made to a publication wherein *Vibrio* sp. strain 5709 isolated from deep sea sediment sample was able to grow at 0°C and produce protease which was active at pH 5 and optimal temperature for activity was 40°C and 45% of the optimal activity was obtained at 20°C (Hamamato et al., 1995. Characterization of a protease from psychrophilic Vibrio sp. strain 5709. Journal of Marine Biotechnology, 2: 219 - 222). However, the optimum growth temperature for this isolate is at 20°C, which can be expensive from the point of energy consumption and practical application in tropical countries. Our culture grows at 30°C, shows optimum activity at 45°C but retains 90% of its activity at 30°C and 50% of activity at 15°C and 60°C.
**(vii)** The biosynthesis and properties of an extracellular metalloprotease from the Antarctic marine bacterium *Sphingomonas paucimobilis* were investigated by Turkiewicz et al. (Journal of Biotechnology, 70, 219-222, 1999).

### OBJECTS OF THE INVENTION

The main object of the present invention is to isolate a novel marine strain of the fungus *Aspergillus* sp., from deep-sea sediments collected at a depth of ∼5000m in the Central Indian basin.

Another main objective of the present invention is to provide a process for production of protease using the marine strain of the fungus *Aspergillus* sp. MTCC 5102 for possible use in industries wherever protease is required which obviates drawbacks as detailed above. Yet another objective of the present invention is to provide a method for growing the said fungus on a large scale using inexpensive commonly available spray-dried dairy whitener as the nitrogen source and glucose as the carbon source in a medium prepared with distilled water or seawater at pH 7 or 8.5.

Still another objective of the present invention is to provide a culture having protease activity at a broad range of pH from 6 to 11.

Still another objective of the present invention is to provide an alkaline protease, which is active at a broad range of temperatures from 15 to 45°C.

Still another objective of the present invention is to provide a culture, which grows at 5°C or 30°C.

One more objective of the present invention is to provide a culture showing lipase activity in the culture supernatant.

### SUMMARY OF THE INVENTION

Thus, the present invention particularly relates to production of alkaline protease for various industries and especially for "enzyme detergents" by a novel fungus Aspergillus sp. deposited in the microbial type culture collection (MTCC) of Institute of Microbial Technology, Chandigarh, India, under the accession number MTCC 5102. The said fungus can be grown in conventional media with commercial brands of milk powder using distilled water at pH 7.0 at room temperature (28°C±2°C). However, the said fungus grows well in seawater media too and also at 5°C. Further, the protease enzyme produced by this fungus acts equally well in the pH range of 6 to 11. It shows temperature optimum at 45°C but almost 90% of the activity is present at 30°C, 50% of the activity at 15°C and at 60°C. The enzyme is thermostable up to 45 min at 45° C. The enzyme is not inhibited by EDTA and thus is not a metalloprotease. Its activity is totally inhibited in the presence of phenylmethylsulfonyl fluoride (PMSF) suggesting the protease enzymes to be a serine protease.

### DETAILED DESCRIPTION OF THE INVENTION

Present invention particularly relates to production of alkaline protease for various industries and especially for "enzyme detergents" by a fungus *Aspergillus sp.* deposited in the microbial type culture collection of Institute of Microbial Technology, Chandigarh, India, under the accession number MTCC 5102. The said fungus can be grown in conventional media with commercial brands of milk powder using distilled water at pH 7.0 at room temperature (28°C±2°C). However, the said fungus grows well in seawater media too and also at 5°C. Further, the protease enzyme produced by this fungus acts equally well in the pH range of 6 to 11. It shows temperature optimum at 45°C but almost 90% of the activity is present at 30°C, 50% of the activity at 15°C and at 60°C. The enzyme is thermostable up to 45 min at 45°C.

A process according to an aspect of the present invention for producing protease enzyme comprises growing of a marine strain of the fungus *Aspergillus* sp. MTCC 5102 in conventional media containing carbon and nitrogen source besides an additional organic nitrogen source for a period of 4-6 days to get the protease enzyme, said protease enzyme being active at pH 6-11 and temperature of 15°C to 60°C.

The alkaline protease shows optimum activity at 42-47°C but about 90% of the maximum activity is present at 30°C and about 50% of the activity is seen at 15°C and 60°C.

The alkaline protease produced by the said fungus is stable at 45°C for at least 10 min showing 100% activity and about 50% of the maximum activity is retained after 10 minutes treatment of the enzyme at 60°C.

The alkaline protease produced by the said fungus is stable at 45°C for a period of 45 minutes.

The alkaline protease produced by the said fungus is not a metalloprotease.

The low temperature active alkaline protease thus obtained with the above mentioned characteristics can be used as detergent additive, for dehairing of hides, in food industries, for processing of waste feathers, recovery of silver from gelatine-coated X-ray films and treatment of industrial and domestic waste and other similar applications wherever required.

### BRIEF DESCRIPTION OF ACCOMPANYING DRAWINGS

- **Figure 1A**: Represents alkaline protease production by the MTCC 5102 using different nitrogen and carbon sources.
- **Figure 1B**: Shows protease production in media prepared with distilled water or sea water adjusted to different pHs. The protease activity was measured at the same pHs at which the isolate was grown.
- **Figure 1C**: Shows protease production by cultures at 4°C and 30°C at different intervals.
- **Figure 2**: Represents protease activity under various pH ranging from 6-12 using Azocasein as the substrate.
- **Figure 3**: Represents protease activity in the temperature range of 5 to 75°C.
- **Figure 4A**: Represents thermostability of protease enzyme for 10 minutes when incubated at various temperatures
- **Figure 4B**: Represents stability of protease at 45°C for various time intervals.
- **Figure 5A**: Represents protease activity with reference to assay time.
- **Figure 5B**: Shows activity at different concentrations of enzyme protein.
- **Figure 5C**: Shows enzyme activity at different concentrations of the substrate Azocasein.
- **Figure 6**: Shows destaining of fresh as well as 48 h old blood stains on cotton fabric using culture filtrate with protease activity.

### BRIEF DESCRIPTION OF ACCOMPANYING TABLES

**Table 1** Gives the enzyme activity at different temperature.
**Table 2** Shows the protease activity in the presence of various detergents
**Table 3** Shows the effect of EDTA on the enzyme activity
**Table 4** Shows the comparative data about the enzyme activity as compared to the known one.

The organism given in the present invention is a Deuteromycete fungus isolated from deep-sea sediments collected from 5494 m depth in the Central Indian Basin and is identified to be *Aspergillus* sp. having the accession number MTCC 5102 and is deposited in the Institute for Microbial Technology, Chandigarh, India. The fungal colonies appear granular, light yellow-green to deep yellow-green in color, uniseriate, conidial heads globose, conidia globose and echinulate. The said fungus can be grown in malt extract broth containing malt extract and peptone. The fungal mat grown this way may be macerated and used as a starter inoculum for the experimental cultures containing glucose at a concentration of 1% (w/v). Casein, spray-dried dairy whitener, soybean meal, molasses or corn steep liquor at 1% concentration can be used as the nitrogen source. The pH of the medium can be adjusted to 7.0 or 9.0. This medium can be prepared with distilled water or sea water. The culture can be grown at 5°C or 30°C. The cultures are grown as shallow static cultures for 4-6 days. The cell-free clear supernatant is used as the source of alkaline protease. Protease activity is measured using Azocasein (Sigma Chemicals, Mo, USA) as substrate.

The said fungus *Aspergillus* sp. MTCC 5102 is capable of growth and production of protease enzyme in simple, inexpensive medium containing glucose as a carbon source and spray-dried dairy whitener as an organic nitrogen source besides sodium nitrate as an inorganic nitrogen source. The culture is fast-growing and by following our method of inoculation, maximum enzyme production can be obtained by day 6. The said fungus grows at 5 °C also. The said fungus produces protease which shows activity at a broad pH range of 6-11 and with temperature optimum at 45°C. The commercial alkaline proteases such as Alcalase ®, Esperase ® and Savinase ® have temperature optima at 60°C. Our culture shows 90% of the maximum protease activity at 30°C and about 50% of the maximum activity at 15°C and 60°C. The protease enzyme retains 90% of its optimum activity after incubating at 45°C for 45 min.

### EXAMPLES

The following examples are given by way of illustrations of the present invention and therefore, should not be construed to limit the scope of the present invention.

### EXAMPLE 1

The said fungus *Aspergillus* sp. MTCC 5102 is grown in malt extract broth containing 2% malt extract and 0.3% peptone in sea water. The 7-day old fungal mat obtained this way was macerated and used as a starter inoculum for the experimental cultures containing glucose or cellulose at a concentration of 1% (w/v). Casein, spray-dried dairy whitener, soybean meal, molasses or corn steep liquor at a 1% concentration was used as the organic nitrogen source. The Czapek Dox broth to which the above mentioned nitrogen - containing substrates were added independently, contained 10 g glucose, 2g NaNO₃, 1g K₂HPO₄, 0.5 g MgSO₄, 0.5 g KCl and 0.01 g Fe₂SO₄ L⁻¹ medium. The pH of the medium was adjusted to 7.0 or 9.0. This medium was prepared with distilled water or sea water. The cultures were grown as shallow static cultures for 4-6 days. The cell-free clear supernatant obtained after filtration through GF/F filters followed by 0.22 µm Nuclepore filters was used as the source of alkaline protease. Protease activity was measured using Azocasein (Sigma Chemicals, Mo, USA) at 2% concentration. Azocasien Units (ACU) released at various temperatures and pH combinations were measured at 440 nm and expressed as an increase in absorbance by 0.001 in 1 minute (Alfredsson, G.A,, Gudmundsson H.M., Xiang J.Y., Kristjansson M.M. 1995, Subtilisin-like serine proteases from psychrophilic marine bacteria. J. Mar. Biotech. 3: 71-72). Substrate and enzyme blanks were maintained for all the assays. All the assays were run in triplicates.

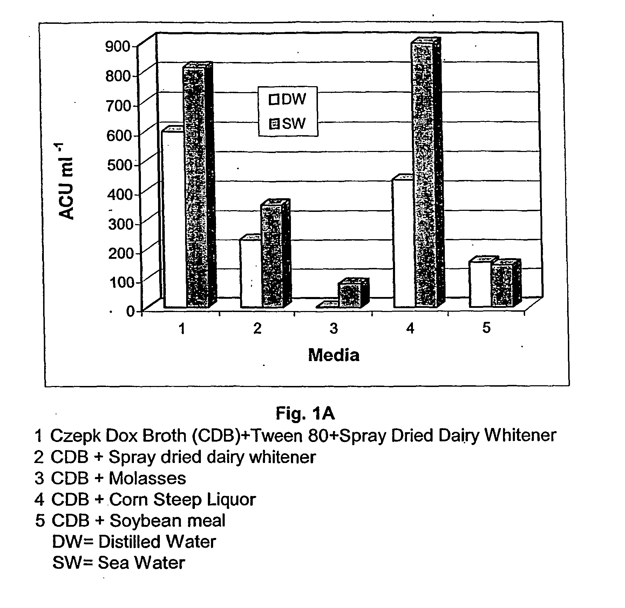

Accordingly Figure 1A of the drawings accompanying these specifications shows comparative production of protease in media containing various carbon and nitrogen sources. The results indicate that protease production is comparable in media prepared with distilled water and sea water. Corn steep liquor appears to be the best substrate for production of protease but it imparts a dark brown color to the culture filtrate and hence was not used for further experiments.

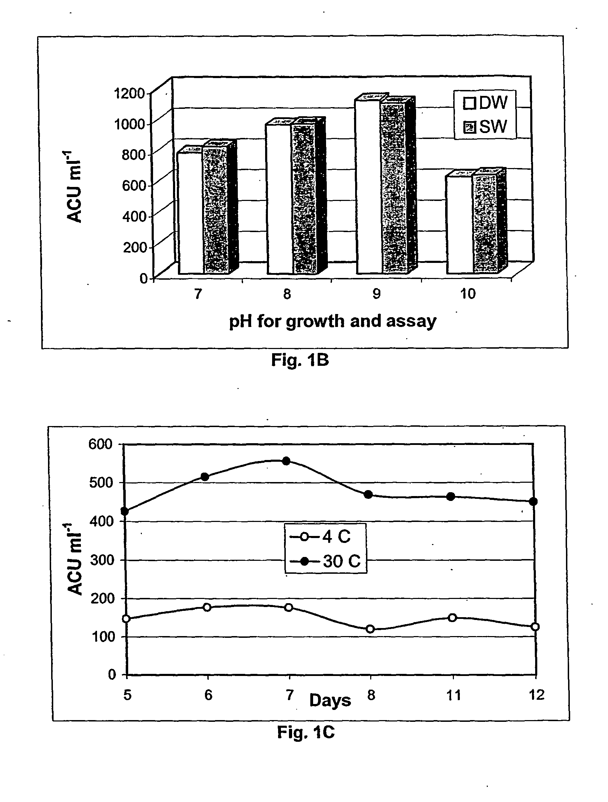

Accordingly Figure 1B of the drawings accompanying these specifications shows comparative production of protease using spray-dried dairy whitener in media prepared either with distilled water or sea water and adjusted to different pHs. The protease activity was assayed at the same pHs. The maximum enzyme activity was observed at pH 9.0 when used for growth as well as enzyme assay.

Accordingly Figure 1C of the drawings accompanying these specifications shows comparative production of protease using spray-dried dairy whitener and incubated at 4°C and 30°C for different time intervals.

### EXAMPLE 2

The said fungus *Aspergillus* sp. MTCC 5102 was grown as described in Example 1 and the clear supernatant was used for estimating the optimum pH for protease activity at 30°C using Azocasein as a substrate. The effect of pH on the reaction was assessed by incubating the reaction mixture containing phosphate buffer at pH 6-8, sodium borate buffer at pH 8.5-9.5 or sodium bicarbonate buffer at pH 10-12. Protease activity was measured as described in Example1.

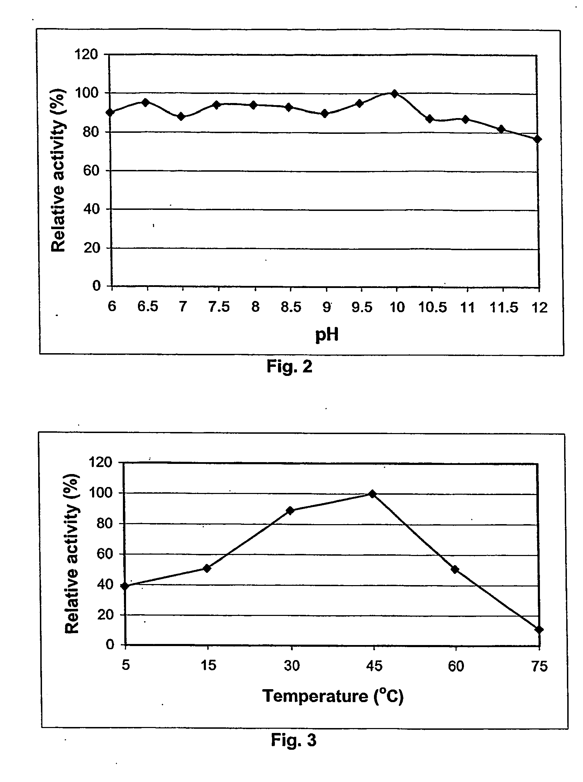

Accordingly, Figure 2 of the drawings accompanying these specifications shows 90-100 % of activity between pH 6 and 11. About 80% of the maximum activity was seen at pH 12.

### EXAMPLE 3

The said fungus *Aspergillus* sp. MTCC 5102 was grown as described in Example 1 and the clear supernatant was used for monitoring the optimum temperature for protease activity at pH 10.0 using Azocasein as the substrate. The effect of temperature on the reaction was assessed by incubating the reaction mixture at different temperatures in the range of 5°C to 75°C. Protease activity was measured as described in Example 1.

Accordingly, Figure 3 of the drawings accompanying these specifications shows optimum activity of protease at 45°C, but about 90% of the maximum activity was observed at 30°C and about 50% of the activity was retained at 15°C and at 60°C. Table 1 shows the enzyme activity at different temperature.

**Table 1**

| **Temperature** | **ACU (Azocasein units)** | **% activity** |
|---|---|---|
| **5** | **460** | **39** |
| **15** | **606** | **51** |
| **30** | **1060** | **89** |
| **45** | **1187** | **100** |
| **60** | **600** | **50** |
| **75** | **132** | **11** |

### EXAMPLE 4

The said fungus *Aspergillus* sp. MTCC 5102 was grown as described in Example 1 and the clear supernatant was used for studying the thermostability of the enzyme preparation. This was monitored by incubating the enzyme for 10 minutes at different temperatures (30, 40, 50, 60, 70, 80 and 90°C) before rapidly cooling in ice and carrying out the activity measurement at 30°C as described in the Example 1.

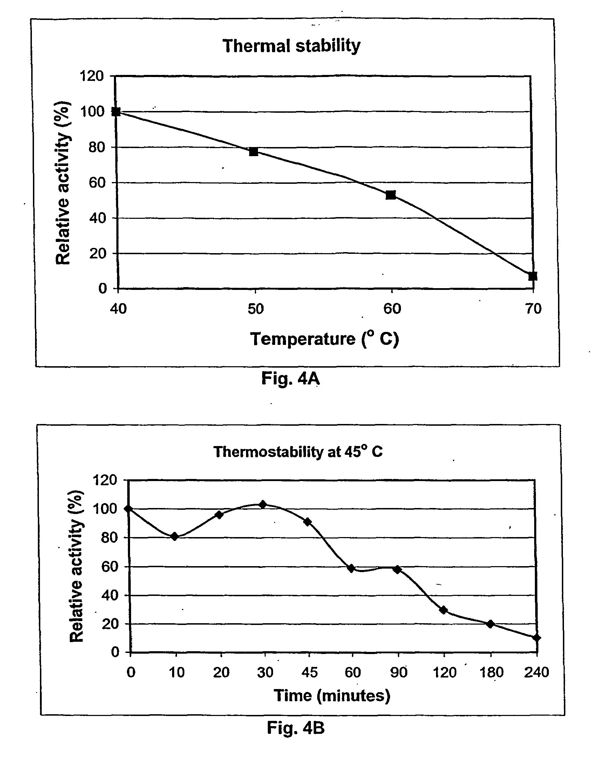

Accordingly, Figure 4A of the drawings accompanying these specifications shows that for at least 10 min the enzyme retained about 90 % of the activity at 40°C and 78 % of the activity at 50°C.

Accordingly, Figure 4B of the drawings accompanying these specifications shows that the enzyme is stable for at least 45 minutes at 45°C.

### EXAMPLE 5

The said fungus *Aspergillus* sp. MTCC 5102 was grown as described in Example 1 and the clear supernatant was used for studying the optimum time of incubation and enzyme and substrate concentrations for assaying protease activity recorded was between 30 to 60 minutes. The time of incubation was monitored by incubating the reaction mixture containing the enzyme and substrate at optimum temperature of 45°C and pH for various time intervals, ranging from 30 to 120 minutes.

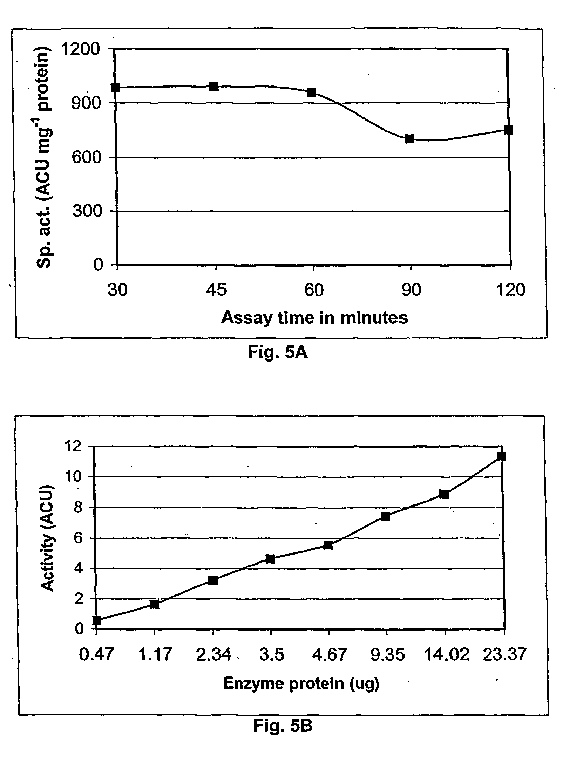

Accordingly, Figure 5A of the drawings accompanying these specifications shows that the enzyme showed optimum activity between 30 and 60 minutes.

Accordingly, Figure 5B of the drawings accompanying these specifications shows that the enzyme showed increasing activity with increasing concentrations of enzyme.

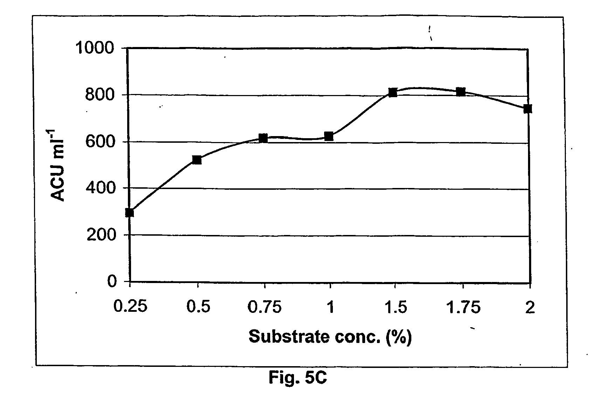

Accordingly, Figure 5C of the drawings accompanying this specification show that the enzyme showed maximum activity at a substrate concentration of 1.5% and above.

### EXAMPLE 6

The said fungus *Aspergillus* sp. MTCC 5102 was grown as described in Example 1 and the clear supernatant was used for performing the cleaning ability of the protease. Fresh and 48 h- old blood stains on cotton fabric were treated for 30 min with culture filtrate at room temperature (30° C). An enzyme dosage of 85 ACU/ml was used for this purpose.

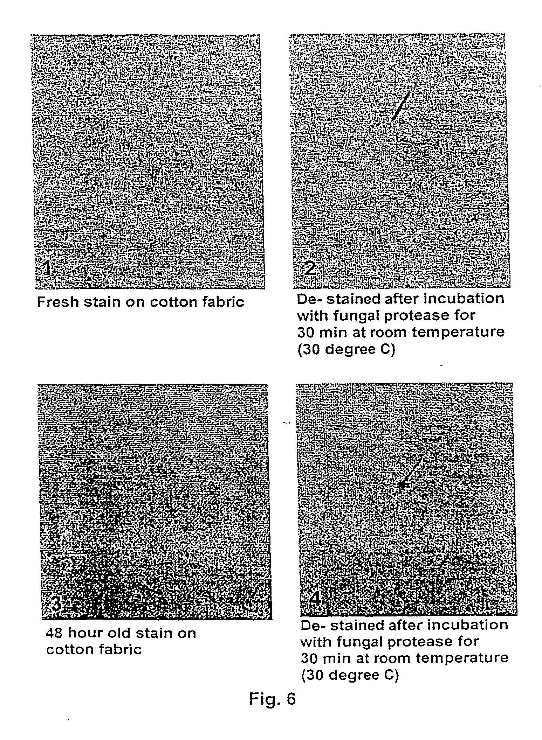

Accordingly, Fig. 6 of the drawings accompanying this specification shows that both fresh as well as old stains of blood are totally removed on treatment with the culture filtrate. The stains in the control treatment, without enzyme incubation did not disappear.

### EXAMPLE 7

The said fungus *Aspergillus* sp. MTCC 5102 was grown as described in Example 1 and the clear supernatant was used for testing the stability of the protease activity in the presence of commercially available detergents. If the enzyme is used as an additive to the detergents it should remain active in their presence and therefore this test was performed. The enzyme was assayed at its optimum temperature of 45°C and as well as at the room temperature (30°C).

Accordingly, Table 2 accompanying this specification shows that when assayed at room temperature most of the activity was retained whereas, at 45°C in some cases, there was a reduction in the enzyme activity.

**Table 2**

| **Detergents added** | **% Azocasein Units** | |
|---|---|---|
| | **Assayed at 45°C** | **Assayed at 30°C** |
| **Detergent 1** | **51** | **91** |
| **Detergent 2** | **78** | **85** |
| **Detergent 3** | **68** | **64** |
| **Detergent 4** | **56** | **62** |
| **Detergent 5** | **58** | **161** |
| **Detergent 6** | **118** | **73** |

### EXAMPLE 8

The said fungus *Aspergillus* sp. MTCC 5102 was grown as described in Example 1 and the clear supernatant was used for testing whether the protease enzyme was serine or metalloprotease. For this purpose the enzyme was assayed in the presence of 50 mM ethylenediamine tetraacetic acid (EDTA), 2 mM phenylmethylsulfonyl fluoride (PMSF) and 200 µM mercuric chloride (HgCl₂).

Accordingly, Table 3 accompanying this specification shows that when assayed in the presence of EDTA there was no inhibition of activity indicating that it is not metalloprotease whereas inhibition of the activity in the presence of PMSF indicated that the enzyme is serine protease. The enzyme is not inhibited in its activity in the presence of HgCl₂.

**Table 3**

| **Addition during assay** | **Residual specific activity (%)** | **% inhibition** |
|---|---|---|
| **Control** | **100** | **0** |
| **EDTA (50 mM)** | **94** | **6** |
| **PMSF (2 mM)** | **0** | **100** |
| HgCl₂ (200 µM) | **86** | **14** |

Though, the disadvantages encountered in the various prior art have been given, yet the applicants substantiate the advantages of the present invention over the prior art by providing a summary of the enzyme activity produced by various strains is shown in Table 1.

**Table 4**

| **Reference** | **Organism** | **Optimum pH** | **Optimum temperature** | **Advantages** | **Disadvantages** |
|---|---|---|---|---|---|
| Takami et al, 1990 (pub.) | *Bacillus sp.* | 11-12 | 70°C | High keratinolytic & elastolytic activities | High optimum temperature for enzyme activity |
| Beck et al. 1988 (patent) | *Paecilomyce s maraquandi* | 7-10.5 | 45°C | Recommended as an additive to detergent along with low temperature active protease from some other source | It is to be grown in carbon at pH 9.2 to produce alkaline protease |
| Liu et al 1988 (patent) | *Nocardiopis dassonvillei* | 8-9 | 60°C | Recommended as an additive to detergent as its activity is not inhibited in the presence of detergent | It is to be grown in carbon and nitrogen sources at 8-10% concentration at alkaline pH values and in the temperature range of 20-30°C |
| Suzuki *&* Odagam i 1997 (publica tion) | *Alteromonas haloplanktis* | 9 | 20°C | About 85% of the activity also observed at 15°C | Only less than 20% of the activity above 20°C, thus it is extremely heat liable. |
| Banerje e & Bhattac harjee 1992 (pub.) | *Rhizopus oryzae* | 3-4 | 60°C | Broad pH range for its activity | Optimum pH range is too high to use it as detergent additive |
| Kun-Hee et al 1999 (pub.) | *Azospirillum sp* | 8.5 | 45°C | The protease is highly stable at temperature below 30°C | The culture does not grow at 37°C. Therefore raising the culture at low temperature may not be cost effective |
| Hamant o et al 1995 (pub.) | *Vibrio sp* | 5 | 40°C | The bacterium grows at 0°C | Optimum growth at 20°C which may become cost prohibitive |
| **OUR PATENT** | *Aspergillus* | 6-12 | 45°C | 50% of the maximum activity is seen at 15°C and 60°C. As the enzyme is active at broad pH and temperature range, it can be used as additives to detergent without mixing other enzymes as claimed in the patent by Beck et al. The enzyme destains blood strains by incubating for 30 minutes at 30°C (room temperature). The fungus produces protease in an inexpensive nitrogen source like dairy whitener at 0.25%. the fungus grows equally well with distilled media or media produced with sea water and produces equal amount of protease. | |

**The main advantages of the present invention are:**
1. The marine isolate of the fungus *Aspergillus* sp. MTCC 5102 can be grown on a large-scale using inexpensive, readily available raw material, namely spray-dried dairy whitener as an additional nitrogen source in the medium to induce protease production. The medium can be prepared using distilled water or sea water.
2. The said fungus can also produce alkaline protease in the presence of other organic nitrogen sources such as corn steep liquor and soybean meal.
3. The pH of the medium can be adjusted to either 7 or 8.5 for the production of protease.
4. The culture can be incubated as static cultures at 30°C or 5°C for production of protease enzyme.
5. The said fungus produces maximum amount of protease within 4-6 days.
6. Protease thus produced shows maximum activity at pH 10 but around 90 to 95% of the maximum activity is observed between pH 6 and 11.
7. Alkaline protease thus produced by the said fungus shows 100% activity at 45°C but about 90% of the maximum activity is present at 30°C and about 50% of the activity is seen at 15°C and 60°C.
8. Alkaline protease of the said fungus is thermo-stable up to 45°C for 10 minutes by retaining 100% the activity and about 50% of the activity is retained at 60°C.
9. Alkaline protease of the said fungus is thermo-stable up to 45 minutes at 45°C.
10. The alkaline protease activity of the said fungus is not inhibited by EDTA and thus is not a metalloprotease and this makes it more desirable in detergent industries.

## Claims

1. A fungus *Aspergillus sp.* deposited in the Microbian Type Culture Collection and Gene bank (MTCC) of Institute of Microbial Technology, Chandigarh, India, under the accession number MTCC 5102.

2. A fungus as claimed in claim 1, wherein the said fungus is:
a) a deuteromycete fungus;
b) it appears granular, light yellow-green to deep yellow-green in color in malt extract agar plate;
c) conidiophores are uni-seriate, conidial heads are globose and echinulate, and
d) grows in the sea water and distilled water with carbon and nitrogen source in a pH range of 7.0-9.0 and temperature range of 5° to 30°C,

3. The fungus claimed in claim 1, wherein it can be grown in distilled water containing carbon and nitrogen source with pH of about 7.0 and temperature of about 30°C.

4. The fungus claimed in claim 1, wherein it can be grown in seawater containing carbon and nitrogen source with pH of about 9.0 and temperature of about 5°C.

5. A process for producing protease enzyme, wherein said protease enzyme is active at pH 6-11 and temperature of 15°C to 60°C, said process comprising the steps of:
a) growing MTCC 5102 in water as culture medium containing malt extract and a nitrogen source to obtain fungal mat;
b) macerating the fungal mat to obtain a starter culture;
c) adding the starter culture to the experimental medium with a pH range of 7.0 to 9.4;
d) allowing the culture to grow for 4 to 6 days as shallow static culture, and
e) filtering the cell free clear supernatant solution obtained from step (d) to obtain alkaline protease.

6. The process as claimed in claim 5, wherein the fungus is *Aspergillus sp.* bearing international deposition number MTCC 5102 having the following characteristics:
a) a deuteromycete fungus;
b) it appears granular, light yellow-green to deep yellow-green in color in malt extract agar plate;
c) conidiophores are uni-seriate, conidial heads are globose and echinulate, and
d) grows in the sea water and distilled water with carbon and nitrogen source in a pH range of 7.0-9.0 and temperature range of 5° to 30°C.

7. The process as claimed in claim 5, wherein the fungus can be grown in distilled water containing carbon and nitrogen source with pH of about 7.0 and temperature of about 30°C.

8. The process as claimed in claim 5, wherein the said fungus can be grown in seawater containing carbon and nitrogen source with pH of about 9.0 and temperature of about 5°C.

9. The process as claimed in claim 5, wherein the culture media is water mixed with about 0.3% peptone and about 2.0% of malt extract.

10. The process as claimed in claim 5, wherein the experimental medium in step (c) comprises Czapek Dox broth to which are added glucose or cellulose at a concentration of 1% (w/v), casein, spray-dried dairy whitener, soybean meal, molasses or corn steep liquor independently at 1%.

## Patentansprüche

1. Pilz *Aspergillus sp.,* der bei der Mikrobenartkultursammlung und Genbank (MTCC) des Instituts für Mikrobielle Technologie, Chandigarh, Indien, unter der Zugangsnummer MTCC 5102 hinterlegt ist.

2. Pilz nach Anspruch 1, wobei der Pilz:
a) ein Deuteromycota-Pilz ist;
b) körnig und in einer Malzextrakt-Agar-Platte in der Farbe hellgelbgrün bis tiefgelbgrün erscheint;
c) uniseriate Konidiophore aufweist, und die Köpfe kugelförmig und echinulat sind und
d) in Meerwasser und in destilliertem Wasser mit einer Kohlenstoff- und Stickstoffquelle in einem pH-Bereich von 7,0-9,0 und einem Temperaturbereich von 5° bis 30°C wächst.

3. Pilz nach Anspruch 1, wobei er in destilliertem Wasser, das eine Kohlenstoff- und Stickstoffquelle enthält, mit einem pH-Wert von etwa 7,0 und einer Temperatur von etwa 30°C gezüchtet werden kann.

4. Pilz nach Anspruch 1, wobei er in Meerwasser, das eine Kohlenstoff- und Stickstoffquelle enthält, mit einem pH-Wert von etwa 9,0 und einer Temperatur von etwa 5°C gezüchtet werden kann.

5. Verfahren zum Herstellen eines Protease-Enzyms, wobei das Protease-Enzym bei einem pH-Wert von 6-11 und einer Temperatur von 15°C bis 60°C aktiv ist, und das Verfahren die Schritte aufweist:
a) Züchten von MTCC 5102 in Wasser als Kulturmedium, das Malzextrakt und eine Stickstoffquelle enthält, um einen Pilzboden zu erhalten;
b) Aufquellen des Pilzbodens, um eine Starterkultur zu erhalten;
c) Hinzufügen der Starterkultur zu dem Versuchsmedium mit einem pH-Bereich von 7,0 bis 9,0;
d) Wachsen lassen der Kultur für 4 bis 6 Tage als flache stationäre Kultur, und
e) Filtern der zellfreien klaren überstehenden Lösung, die aus Schritt (d) erhalten wurde, um eine alkalische Protease zu erhalten.

6. Verfahren nach Anspruch 5, wobei der Pilz *Aspergillus sp.* ist, der die internationale Hinterlegungsnummer MTCC 5102 trägt und die folgenden Merkmale aufweist:
a) er ist ein Deuteromycota-Pilz;
b) er erscheint körnig in einer Malzextrakt-Agar-Platte in der Farbe hellgelbgrün bis tiefgelbgrün;
c) die Konidiophore sind uniserat, die konidialen Köpfe sind kugelförmig und echinulat, und
d) er wächst in Meerwasser und in destilliertem Wasser mit einer Kohlenstoff-und Stickstoffquelle in einem pH-Wert-Bereich von 7,0-9,0 und einem Temperaturbereich von 5° bis 30°C.

7. Verfahren nach Anspruch 5, wobei der Pilz in destilliertem Wasser, das eine Kohlenstoff- und eine Stickstoffquelle enthält, bei einem pH-Wert von etwa 7,0 und einer Temperatur von etwa 30°C gezüchtet werden kann.

8. Verfahren nach Anspruch 5, wobei der Pilz in Meerwasser, das eine Kohlenstoff-und eine Stickstoffquelle enthält, mit einem pH-Wert von etwa 9,0 und einer Temperatur von etwa 5°C gezüchtet werden kann.

9. Verfahren nach Anspruch 5, wobei das Kulturmedium Wasser vermischt mit etwa 0,3% Pepton und etwa 2,0% Malzextrakt ist.

10. Verfahren nach Anspruch 5, wobei das Versuchsmedium im Schritt (c) eine Czapek-Dox-Brühe enthält, zu der Glucose oder Cellulose in einer Konzentration von 1% (w/v), Kasein, sprühgetrockneter Milchweißmacher, Sojabohnenmehl, Melasse oder Maisquellwasser mit jeweils 1 % hinzugefügt werden.

## Revendications

1. Champignon de l'espèce *Aspergillus,* déposé à la Microbial Type Culture Collection and Gene bank (MTCC) de l'Institute of Microbial Technology, Chandigarh, Inde, sous le numéro d'enregistrement MTCC 5102.

2. Champignon tel que revendiqué dans la revendication 1, dans lequel ledit champignon est :
a) un champignon du groupe des deutéromycètes ;
b) il a un aspect granulaire, une couleur jaune vert clair à jaune vert foncé dans une plaque de gélose avec de l'extrait de malt ;
c) les conidiophores sont unisériés, les têtes conidiales sont globuleuses et échinulées, et
d) il croît dans l'eau de mer et l'eau distillée avec une source de carbone et d'azote dans une plage de pH de 7,0 à 9,0, et une plage de température de 5 à 30°C.

3. Le champignon revendiqué dans la revendication 1, pouvant être cultivé dans de l'eau distillée contenant une source de carbone et d'azote, avec un pH d'environ 7,0 et une température d'environ 30°C.

4. Le champignon revendiqué dans la revendication 1, pouvant être cultivé dans de l'eau de mer contenant une source de carbone et d'azote, avec un pH d'environ 9,0 et une température d'environ 5 °C.

5. Procédé pour la production d'une enzyme de type protéase, dans lequel ladite enzyme de type protéase est active à un pH de 6 à 11 et une température de 15 °C à 60 °C, ledit procédé comprenant les étapes consistant à:
a) cultiver le champignon MTCC 5102 dans de l'eau en tant que milieu de culture, contenant un extrait de malt et une source d'azote pour obtenir une nappe fongique ;
b) faire macérer la nappe fongique pour obtenir une culture starter ;
c) ajouter la culture starter au milieu expérimental avec une plage de pH de 7,0 à 9,0 ;
d) permettre à la culture de se développer pendant 4 à 6 jours sous la forme d'une culture superficielle statique ; et
e) filtrer la solution surnageante limpide sans cellule, obtenue à partir de l'étape (d) pour obtenir une protéase alcaline.

6. Le procédé tel que revendiqué dans la revendication 5, dans lequel le champignon est de l'espèce *Aspergillus* portant le numéro international de dépôt MTCC 5102, ayant les caractéristiques suivantes :
a) champignon du groupe des deutéromycètes ;
b) il a un aspect granulaire, une couleur jaune vert clair à jaune vert foncé dans une plaque de gélose avec de l'extrait de malt ;
c) les conidiophores sont unisériés, les têtes conidiales sont globuleuses et échinulées, et
d) croît dans l'eau de mer et l'eau distillée avec une source de carbone et d'azote dans une plage de pH de 7,0 à 9,0, et une plage de température de 5 à 30°C.

7. Le procédé tel que revendiqué dans la revendication 5, dans lequel le champignon peut être cultivé dans de l'eau distillée contenant une source de carbone et d'azote avec un pH d'environ 7,0 et une température d'environ 30°C.

8. Le procédé tel que revendiqué dans la revendication 5, dans lequel ledit champignon peut être cultivé dans de l'eau de mer contenant une source de carbone et d'azote avec un pH d'environ 9,0 et une température d'environ 5°C.

9. Le procédé tel que revendiqué dans la revendication 5, dans lequel le milieu de culture est de l'eau mélangée avec environ 0,3 % de peptone et environ 2,0 % d'extrait de malt.

10. Le procédé tel que revendiqué dans la revendication 5, dans lequel le milieu expérimental dans l'étape (c) comprend du bouillon de Czapek-Dox auquel on a ajouté du glucose ou de la cellulose à une concentration de 1 % (p/v), de la caséine, un agent blanchissant lyophilisé dérivé du lait, de la farine de soja, de la mélasse ou de la liqueur de macération de maïs, indépendamment, à raison de 1 %.
